# EUROPEAN PATENT APPLICATION

(11) **EP 1 626 089 A1**
(43) Date of publication of application: **15.02.2006**
(21) Application number: 04018824.5
(22) Date of filing: 09.08.2004
(51) Int. Cl.: C12N 15/82, A01H 5/00

(54) **Lipid metabolic and signalling pathways in the epidermis**

(71) Applicant: Max-Planck-Gesellschaft zur Förderung der Wissenschaften e.V., 80539 München (DE); Bayer CropScience GmbH, 65929 Frankfurt (DE)
(72) Inventor: Yephremov, Alexander, 50829 köln (DE); Saedler, Heinz, 50829 Köln (DE); Tietjen, Klaus-Günther, 40764 Langenfeld (DE); Trenkamp, Sandra, 40223 Düsseldorf (DE); Schreiber, Lukas, 53347 Alfter (DE)
(74) Representative: Dehmel, Albrecht

(57) **Abstract**

The present invention relates to the field of genes isolated from Arabidopsis that code for enzymes that are involved in ω-oxidation of very long chain fatty acids, particularly, in conversion of ω-hydroxy fatty acid to fatty aldehyde. The invention also includes methods of producing transgenic plants with an altered fatty acid composition. Furthermore, the present invention relates to assays making use of these enzymes as herbicide targets to identify inhibitors of said enzymes, thereby providing herbicidally active compounds.

## Description

The present invention relates to the field of genes isolated from Arabidopsis that code for enzymes that are involved in ω-oxidation of very long chain fatty acids, particularly, in conversion of ω-hydroxy fatty acid to fatty aldehyde. The invention also includes methods of producing transgenic plants with an altered fatty acid composition. Furthermore, the present invention relates to assays making use of these enzymes as herbicide targets to identify inhibitors of said enzymes, thereby providing herbicidally active compounds.

Very long-chain fatty acids (VLCFA) could be metabolised by three oxidation pathways targeting a carbon in the α-, β- and ω-position in the chain (Bremer and Osmundsen, 1984). The majority of fatty acids are channelled into the β-oxidation pathway in peroxisomes that results in progressive shortening of fatty acids. The endoplasmic reticulum-localised ω-oxidation is generally considered as a minor pathway, which can be activated in case β-oxidation fails to proceed normally. It should be pointed out, however, some fatty acid derivatives are predominantly inactivated through ω-oxidation pathway (e.g. β-methyl-heptadecanoic acid in the heart (Fink *et al*., 1990), leukotriene B4 (LTB4) in human neutrophils (Sumimoto and Minakami, 1990) and rat hepatocytes (Wheelan *et al*., 1999)).

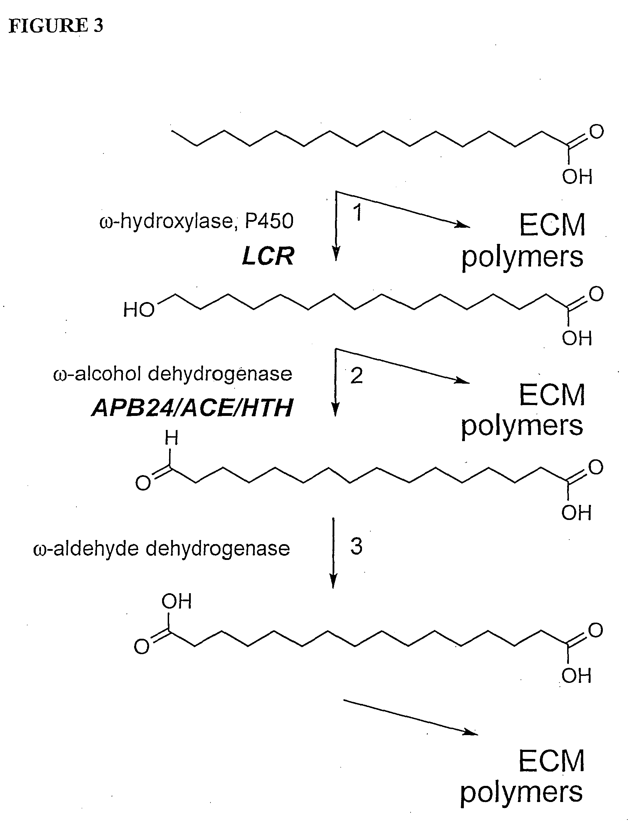

In contrast to the β-oxidation, the ω-oxidation of VLCFAs does not require their activation with coenzyme A (CoA). The ω-oxidation proceeds in three steps (Fig. 3). Firstly, the ω-carbon of VLCFA is oxidised to an alcohol, then to an aldehyde and at last to a carboxylic acid. The resulting product is a very long chain α-,ω-dicarboxylic fatty acid, which has carboxyl groups on both α- and ω-ends. Products of ω-oxidation can be activated into very long chain dicarboxylyl-CoAs esters by dicarboxylyl-CoA synthetase (Vamecq *et al*., 1985). Therefore, when the ω-oxidation is coupled to the β-oxidation, very long chain α-,ω-dicarboxylic fatty acids may be shortened and effectively metabolised. Accordingly, the stimulation of ω-oxidation is linked to induction of peroxisomal proliferation.

The first step of ω-oxidation (Fig. 3), is catalysed by ω-hydroxylases, which belong to cytochrome P450 gene family 4 in mammals, to family 52 in several *Candida* species (Nelson *et al*., 1996) and to family 86 in plants (Benveniste *et al.,* 1998; Werck-Reichhart *et al.,* 2001). At the next two steps (Fig. 3), the very long chain fatty alcohol is oxidised to α-,ω-dicarboxylic fatty acid by action of fatty alcohol:NAD oxidoreductase complex (Lee, 1979; Rizzo *et al*., 1987), which consists of separate proteins: fatty alcohol dehydrogenase (ADH, EC 1.1.1.1) catalysing oxidation of fatty alcohol to fatty aldehyde, and fatty aldehyde dehydrogenase (AldDH, EC 1.2.1.5), catalysing oxidation of fatty aldehyde to α-,ω-dicarboxylic fatty acid (Ichihara *et al*., 1986a; Ichihara *et al.,* 1986b). ADH and AldDH use reduced NADH and oxidised NAD⁺ (nicotinamide adenine dinucleotide) as cofactors, respectively.

In animals, products of ω-oxidation are considered as intermediate metabolites, elevated levels of which in urine (dicarboxylic aciduria) could be used as an indicator of a fatty acid metabolism disorder. In plants, α-,ω-dicarboxylic fatty acids are structural materials that are found mainly in the protective coverings formed by cutin and suberin deposited in the cell walls. Very long chain α-,ω-dicarboxylic fatty acids represent aliphatics nearly unique to suberin (Matzke and Riederer, 1991). Although they were reported, for example, in cutin of *Solanum tuberosum, Spinacia oleracea* and species of *Brassicacea,* they generally comprise a minor proportion of the total cutin monomers (reviewed in (Holloway, 1982)). In contrast, α-,ω-dicarboxylic fatty acids belong to major structural components of suberin comprising up to 33% of monomers (Bernards, 2002; Holloway, 1983).

Suberin deposited in the cell walls in the root endodermis forms a waterproof cylinder called the Casparian strip. It is also detectable in aerial organs in the cells surrounding vascular bundles (bundle sheath). Suberin is a major structural component, which accumulates following wounding to protect plant against pathogens and water loss. Although being an important defence response, formation of suberin barrier may result in premature wilting in cut flowers because stems are no longer able to take up water.

Biochemical evidence for the presence of wound-induced ω-hydroxyfatty acid dehydrogenase required for biosynthesis of α-,ω-dicarboxylic fatty acids in suberin has been provided (Agrawal and Kolattukudy, 1978a; Agrawal and Kolattukudy, 1978b). The enzyme has been purified 600-fold from wound-healing potato (*Solanum tuberosum* L.) tuber disks to near homogeneity, and its molecular weight has been estimated to be about 31.000 Da.

The nucleotide and amino acid sequences of this enzyme remained unknown. No other nucleic acid sequences for ω-hydroxyfatty acid dehydrogenases that are involved in the biosynthesis of extracellular matrix polymers in plants have been provided.

The only genes that encode for very long chain fatty alcohol dehydrogenases involved in ω-oxidation have been isolated from *Candida cloacae* and related yeast species capable of growing on alkanes and fatty acids as sole carbon sources (Vanhanen *et al*., 2000). Physiological function of these enzymes involved in a degradative pathway of fatty acids is apparently quite different from enzymes involved in suberin and cutin production in plants. In databases, plant proteins could be found that show sequence similarities to fatty alcohol dehydrogenases isolated from *Candida* (Vanhanen *et al*., 2000). These proteins, however, are sufficiently distinct from the protein described in this invention. The recent publications describing molecular isolation of the *ADHESION OF CALYX EDGES* (*ACE*) or *HOTHEAD* (*HTH*) gene of Arabidopsis (Araki *et al*., 1998; Araki and Nakatani-Goto, 1999; Krolikowski *et al*., 2003) that is identical to the APB24 gene described in this invention, did not contain an indication or an evidence with regard to function of the protein.

The problem to be solved by the present invention is to provide a transgenic plant having a changed lipid composition. It is a further aim of the present invention to provide a transgenic plant having a changed lipid composition for use in the production of biopolymers on fatty acid basis. It is a further aim of the present invention to provide a transgenic plant having a changed lipid composition for use in the production of therapeutically active agents, cosmetic or pharmaceutical compositions. It is yet a further aim of the present invention to provide a method for selecting inhibitory compounds.

The problem is solved by the subject-matter defined in the claims.

The present invention is further illustrated by the following figures.

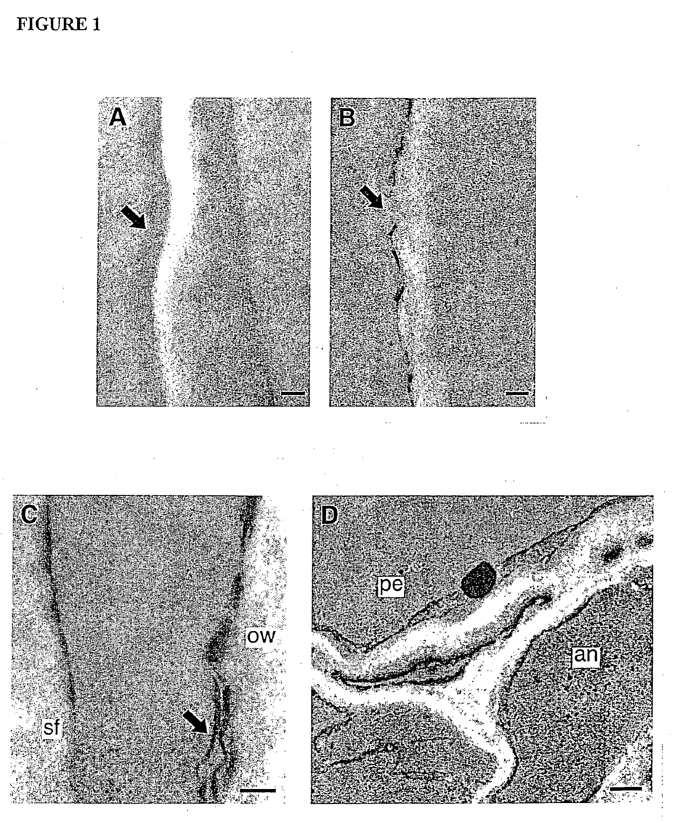

Figure 1. Cuticular defects associated with the loss of the APB24 function.
   To illustrate the mutant phenotype, floral organs, in which the APB24 is strongly expressed, have been inspected with transmission electron microscopy. Scale are 0.1 µm for (A to C), 0.5 µm for (D). (A to D) Cuticle proper (arrows) of septum is seen on electron micrographs as a dark layer above the light cell wall. In mutants, it may be discontinuous (B) or multilayered (C) compared to wild type (A); sf - stamen filament, ow - ovary wall.
   (D) Cell wall fusions between anther an and petal pe.

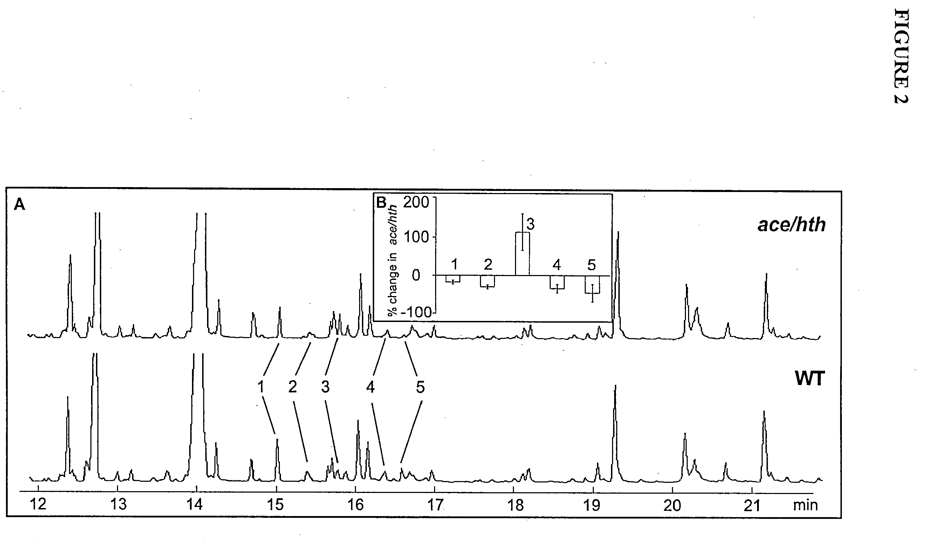

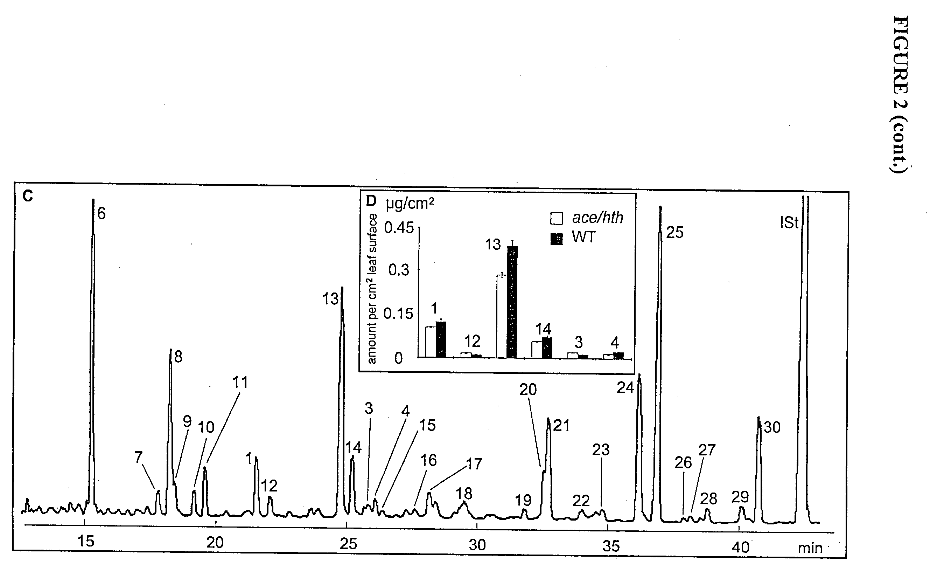

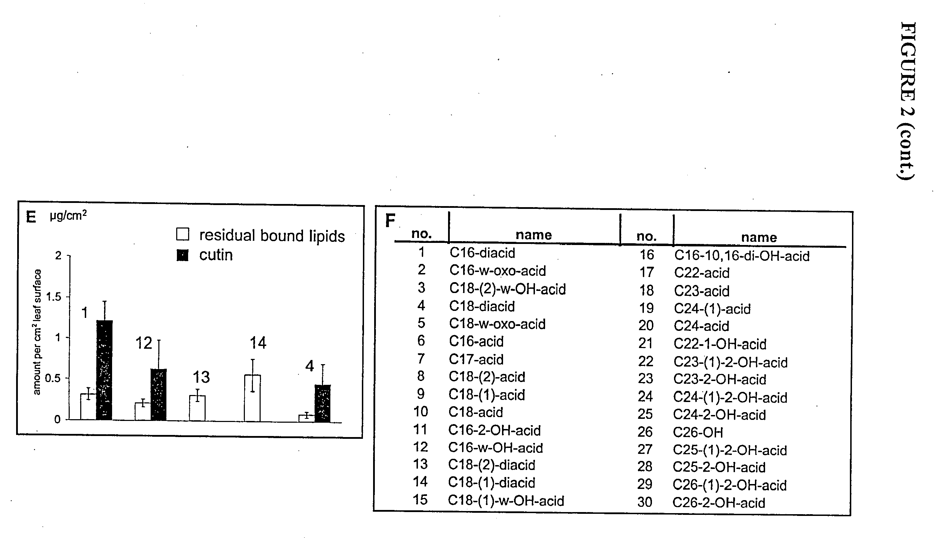

Figure 2. Function of APB24 in plants in biosynthesis of fatty acids.
   Fatty acid metabolites have been analysed using GC-MS.
   (A) Comparative metabolic profiling of total lipids extracted from APB24 mutant and wild type inflorescences.
   (B) Five metabolites (1-5), the accumulation of which is affected in the APB24 mutant, are shown as mean (±SE) percentage changes (n=3).
   (C) Metabolic profiling of residual bound lipids extracted from wild type mature leaves.
   (D) Comparative analysis of ω-fatty acid hydroxylation in APB24 and wild type leaves (n=6 for APB24; n=5 for wild type). Accumulated metabolites are shown as µg per square cm.
   (E) Comparative analysis of leaf residual bound lipids and cutin (n=3) in the close relative of Arabidopsis broccoli. Note that unsaturated α-,ω-dicarboxylic fatty acids are not found in cutin. They may be associated with other components of the epidermal cell wall.
   (F) Peak identities in (A to E).
Figure 3. Role of APB24 in the fatty acid omega-oxidation pathway in plants.

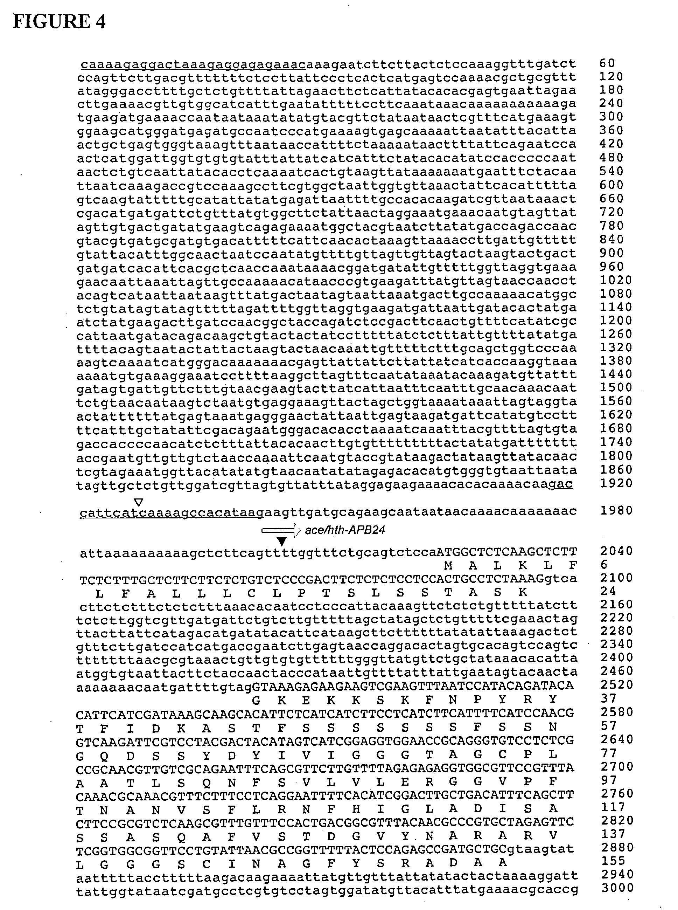

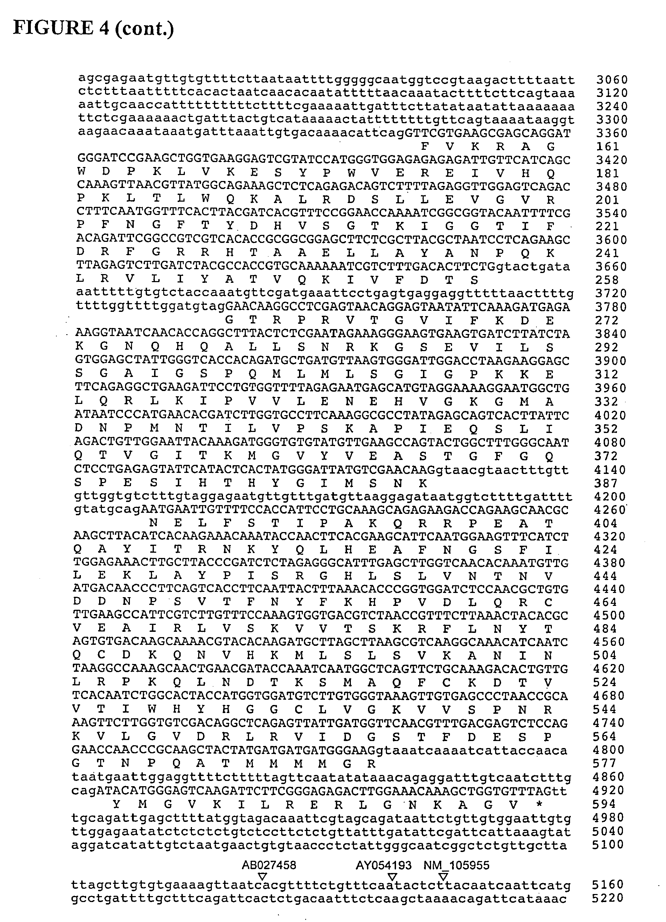

Figure 4. Genomic organization of the APB24 gene
   This figure sets forth the DNA and amino acid sequences of the APB24 gene, including its promoter. The sequences shown here were obtained from Genbank clones (Genbank accession numbers AC008017, AB027458, AY054193, NM105955) and from sequencing of cDNA and promoter clones. Coding sequences are shown in uppercase letters, and 5' and 3' non-coding sequences and introns are shown in lowercase letters. The transcription start sites and polyadenylation sites, as determined from full size cDNA clones (Genbank accession numbers AB027458, AY054193, NM_105955), are indicated by the inverted open triangles. Positions of the transposon insertion is indicated by the inverted black triangles and its orientation is shown by an arrow. Primer sequences used for isolation of the promoter of the APB24 gene are underlined. Numbers to the right represent nucleotide and amino acid positions.

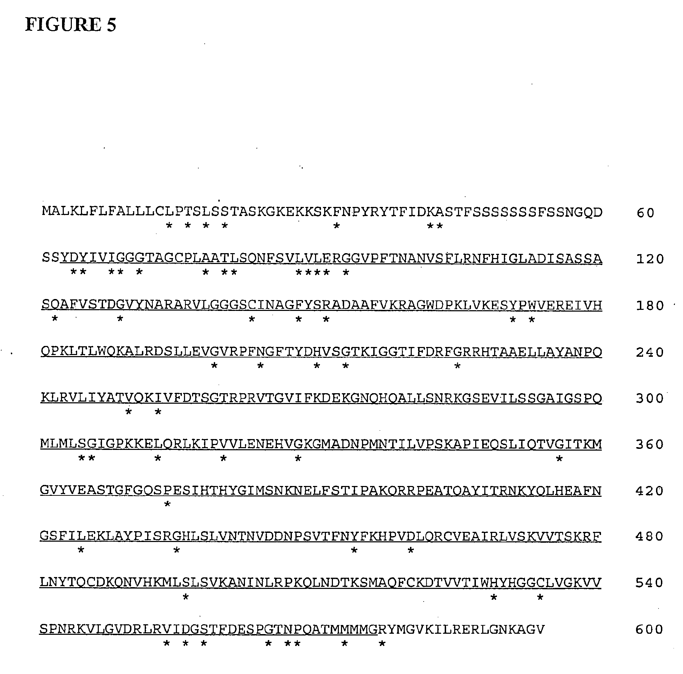

Figure 5. (= SEQ ID NO:2) Amino acid sequence of the APB24 protein
   Amino acid positions from 63 to 580 corresponding to the fragment that exhibits the highest degree of sequence similarity to published glucose-methanol-choline (GMC) oxidoreductases (Cavener, 1992), which contain characteristic sequence domains (Marchler-Bauer *et al*., 2003), are underlined. Amino acids, which are identical in the APB24 protein and all three very long chain fatty alcohol dehydrogenases isolated from *Candida* species (Genbank accession numbers CAB75352, CAB75353, AAS46878), are indicated by asterisks.

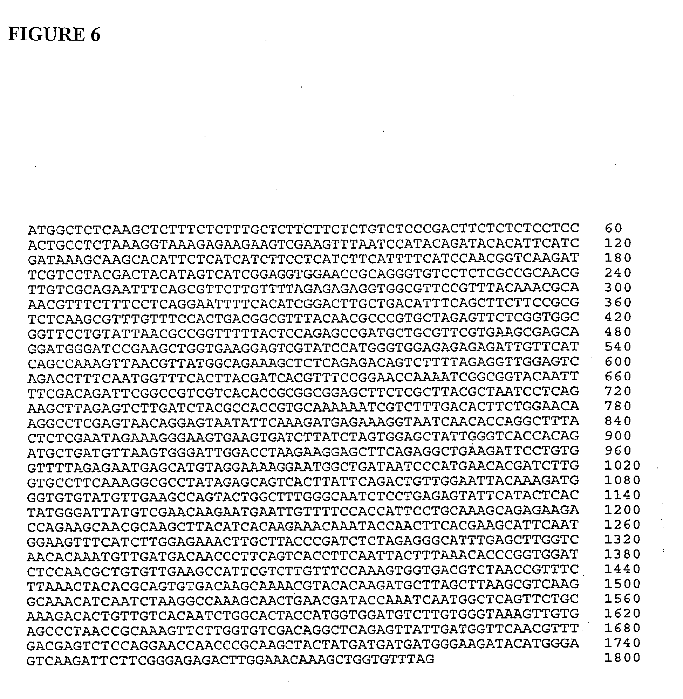

Figure 6. (= SEQ ID NO:1) Coding nucleic acid sequence of the APB24 gene.

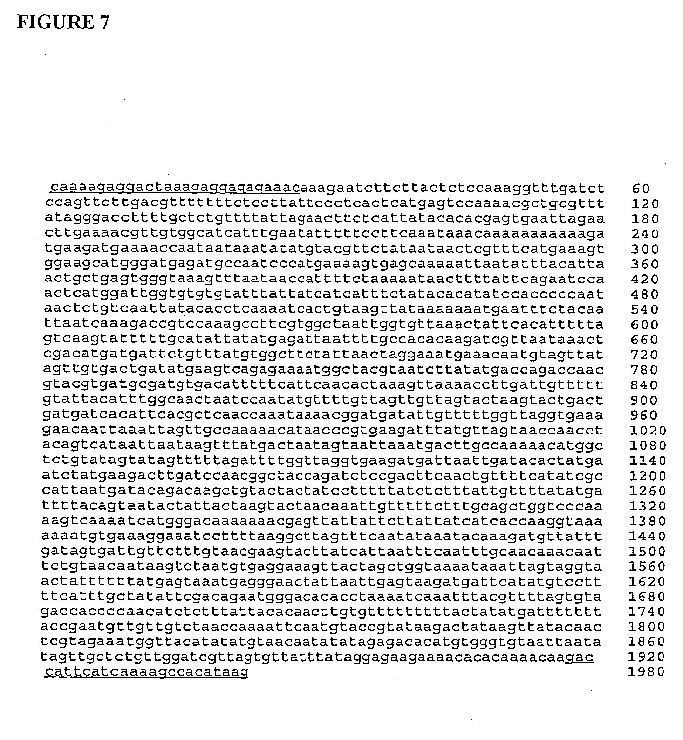

Figure 7. (= SEQ ID NO:3) Nucleic acid sequence of the promoter of the APB24 gene.

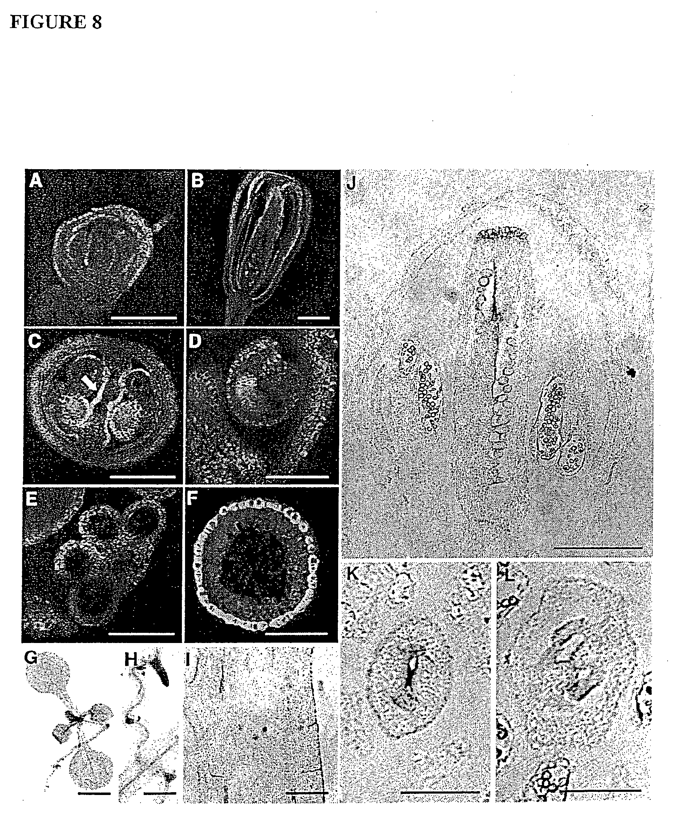

Figure 8. Epidermis specific expression of the APB24 gene.

The term "nucleobase" as used herein refers to heterocyclic nitrogenous bases, particularly purines or pyrimidines (i.e., adenine, cytosine, thymine and guanine), which are natural constituents of nucleic acids where they occur in N-glycosidic linkage with a sugar, particularly a pentose. This term will also include heterocyclic nitrogenous bases, particularly purines or pyrimidines, which constitute unnatural peptide nucleic acids (PNAs) comprising the polyamide backbone with nucleobase side chains. Sequence specific hybridisation of nucleic acids and PNAs occurs through Watson-Crick H-bonding between the complementary nucleobases.

The term "homologue" or "homologous sequence" as used herein refers to nucleic acid or amino acid sequences with significant similarity to the APB24 sequence or parts thereof, whereby these homologous sequences show an activity comparable to the activity of the APB24 gene. In particular, as homologous sequences are considered nucleic acid sequences, which hybridise with the nucleic acid sequence of the APB24 gene or parts of this sequence under stringent or less stringent conditions (about stringent and less stringent conditions see Sambrook *et al.,* Molecular Cloning, Cold Spring Harbour Laboratory (1989), ISBN 0-87969-309-6). An example for stringent hybridisation conditions is: hybridisation in 4 x SSC at 65°C (alternatively in 50% formamide and 4 x SSC at 42°C), followed by several washing steps in 0.1 x SSC at 65°C for one hour. An example for a less stringent hybridisation condition is hybridisation in 4 x SSC at 37°C, followed by several washing steps in 1 x SSC at room temperature. As homologous sequences are furthermore considered nucleic acid or amino acid sequences or parts thereof derived from different plants and showing an activity comparable to the activity of the APB24 gene, wherein said nucleic acid or amino acid sequences or parts thereof show a significant similarity with the nucleic acid and amino acid sequence of the APB24 sequence or parts thereof using the similarity algorithm BLAST (Basic Local Alignment Search Tool, Altschul *et al*., Journal of Molecular Biology 215, 403-410 (1990). As significant similar, as used herein, are considered sequences, which e.g. using standard parameters in Blast-Service of NCBI show a Probability of P < 10⁻⁵, if they are compared with the sequence of the APB24 gene or parts thereof.

The term "homologue" or "homologous sequence" as used herein refers furthermore to nucleic acid or amino acid sequences or parts thereof preferably being identical to the APB24 sequence or parts thereof for at least 60%, more preferably for at least 70% or 80% or 90% and most preferably for at least 95%. The differences to the APB24 sequence or parts thereof can result from deletions, substitutions (like transitions and transversions), additions, insertions and/or inversions.

The term "probe" as used herein refers to nucleic acid sequences or polymer molecules containing nucleobases capable of hybridising to homologous nucleic acid sequences. The said nucleic acid sequences or polymer molecules containing nucleobases should be able to hybridise in a sequence-specific manner to the APB24 sequence or a sequence that is homologous to the APB24 sequence.

The term "derivative" as used herein refers to nucleic acid sequences encoding the APB24 protein or parts thereof, although the nucleic acid sequence differs from the naturally occurring APB24 sequence. Thus, the term derivative refers as well to genomic equivalents carrying introns as to RNA, expressed sequence tags (ESTs), cDNA sequences or to equivalents to the APB24 sequence evolved from the degeneration of the genetic code.

The term "fragment" as used herein refers to the complete amino acid sequence of the APB24 gene, or, preferably, to its fragment from amino acid position 63 to amino acid position 580 as specified in Fig. 5.

The term "vector" as used herein refers to naturally occurring or artificially created constructs for incorporation, propagation, expression or transfer of nucleic acids, e.g. plasmids, phagemids, cosmids, artificial chromosomes, bacteriophages, viruses, retroviruses.

The term "expression system" as used herein refers to any combination of vectors, restriction enzymes, transformation methods, cell extracts, living cells e.g. prokaryotic or eukaryotic cells or organisms for the purpose of endogenous or exogenous expression of genes.

The term "assay" as used herein refers to a method for the determination of the amount of a particular constituent of a mixture that contains a polypeptide encoded by the nucleic acid sequence according to SEQ ID NO:1 or homologous sequences thereof. Thus, the term includes methods for the determination of the biochemical activity of a polypeptide encoded by the nucleic acid sequence according to SEQ ID NO: 1 or homologous sequences thereof.

The term "host cells" as used herein refers to bacterial or eukaryotic cells, which do not naturally contain the nucleic acids or polypeptides encoded by the nucleic acid sequences according to SEQ ID NO:1 or homologous sequences thereof.

The term "transgenic plants" as used herein refers to plants produced by recombinant gene technology and/or microbiological methods but not by usual breeding methods. It is meant that cells of transgenic plants are capable of producing nucleotide or polypeptide molecules that were not produced by corresponding cells of non-transgenic plants prior to transformation. The term "transgenic plants" includes plants obtained through regeneration, vegetative and sexual reproduction.

In the present embodiment, transgenic plants may be produced from any monocotyledonous or dicotyledonous species plants, including, but not limited to, rapeseed, canola, tomato, potato, safflower, sunflower, soybean, peanut, cotton, palm, maize, sorghum, rice, barley and wheat.

The term "transformation" as used herein refers to a process of changing host DNA by transferring foreign DNA into the genome of the host. It includes the transfer of foreign DNA into the host cell followed by integration of said foreign DNA into the DNA of the host. It includes the transfer of foreign DNA into organelles of the host cell, e.g. mitochondria, plastids, or into its nucleus. The transformation as used herein can be achieved by using naked foreign DNA or in combination with other molecules that help breaking, for example, through the cell wall and membranes, transferring foreign DNA in the cell and integrating it into the host DNA.

The term "regeneration" as used herein refers to the development of whole plants from particular cells, tissues or organs. It includes also a classical definition, which means the development of whole plants from undifferentiated cells in vitro in tissue culture using solid or liquid medium supplemented will all nutrient and hormonal components necessary for plant growth.

The term "target nucleic acid" as used herein refers to a nucleic acid sequence according to SEQ ID NO: 1 or homologous sequences thereof and encompasses DNA, RNA, including pre-mRNA and mRNA, transcribed from such DNA, and also cDNA derived from such RNA.

The terms "overexpression" and "overexpressing" as used herein refer to a process of obtaining transgenic plants capable of producing RNA or a polypeptide of interest either in the whole plant or in particular organs or tissues of transgenic plants. It is meant that RNA fragments may be produced in the direct (5' to 3') or in the opposite (3' to 5') orientation.

The term "promoter" as used herein refers to a DNA sequence containing binding sites for proteins involved in the initiation and regulation of transcription from the DNA sequence of interest. The promoter, preferably, is a natural DNA sequence upstream the sequence which encodes a protein. The term "promoter" also refers in this embodiment to synthetic sequences, which are not derived from any organism, that contain binding sites for proteins required for initiation and regulation of transcription. The specific activity of the promoter in transgenic plants, as used herein, refers to a combination of the particular promoter sequences and particular host cells, which are able to provide required proteins. It is meant that promoters can be active in all cells (e.g. ectopic promoters) or in specific cells or tissues (e.g. epidermis or seed specific promoters). It is meant also that promoters can be specifically active only in cells exposed to particular environmental conditions (e.g. water deficit) when suitable transcriptional regulation proteins become available in the cell in response to these conditions.

The terms "extracellular matrix polymers" or "ECM polymers" as used herein refer to polysaccharides, polypeptide, aliphatic and aromatic lipid molecules that are cross-linked in a network outside the cellular membrane of the plant cell. The above mentioned terms shall comprise insoluble materials of cell wall, cuticle, suberin, lignin and sporopollenin.

The term "substrate", as used herein, refers to a chemical substance that can be modified due to enzymatic activity of the polypeptide encoded by the nucleic acid sequence according to SEQ ID NO: 1 or a homologous nucleic acid sequence thereof. It refers but is not limited to ω-hydroxyfatty acids having 8 to 40 carbon atoms that are natural substrates of the polypeptides described herein. Preferably, it refers to ω-hydroxyfatty acids having 14 to 30 carbon atoms, and more preferred to ω-hydroxyfatty acids having 14 to 24 carbon atoms, further to ω-hydroxyfatty acids having 16 to 24 carbon atoms, and further to ω-hydroxyfatty acids having 14 to 20 carbon atoms.

The term "product", as used herein, refers to a chemical substance that can be produced from a substrate due to enzymatic activity of the polypeptide encoded by the nucleic acid sequence according to SEQ ID NO: 1 or a homologous nucleic acid sequence thereof. Preferably, it refers but is not limited to ω-oxofatty acids having 8 to 40 carbon atoms that are natural products of the polypeptides described herein. Preferably, it refers to ω-oxofatty acids having 14 to 30 carbon atoms, and more preferred to ω-oxofatty acids having 14 to 24 carbon atoms, further to ω-oxofatty acids having 16 to 24 carbon atoms, and further to ω-oxofatty acids having 14 to 20 carbon atoms.

The term "inhibitor", as used herein, refers to a chemical substance that inactivates the enzymatic activity of the polypeptides encoded by the nucleic acid sequence according to SEQ ID NO:1 or a homologous nucleic acid sequence thereof. The inhibitor may function by interacting with a cofactor of the enzyme, the substrate of the enzyme, or preferably by interacting directly with the enzyme.

The terms "biological activity" or "enzymatic activity", as used herein, refer to the natural enzymatic activity of the polypeptides encoded by the nucleic acid sequence according to SEQ ID NO:1 or a homologous nucleic acid sequence thereof, wherein said enzymatic activity may be directly or indirectly measured by methods known by the skilled artisan, and wherein the measurement allows to estimate the catalytic activity of said enzymatically active polypeptides.

The terms "herbicide" or "herbicidal compound", as used herein, refer to inhibitors as defined above, and which may be used to kill or suppress the growth of plant cells, tissues or a whole plant.

The present invention relates to the APB24 gene which encodes a very long chain fatty alcohol dehydrogenase involved in the biosynthesis of α-,ω-dicarboxylic fatty acids naturally required for the proper formation of the cuticular layer of the cell wall in plants and the cuticle.

Based on this finding, the present invention refers to methods for producing plants with modified properties, comprising stable integration of at least of the nucleic acid sequence according to SEQ ID NO:1, or a homologous nucleic acid sequence thereof, or of a fragment or derivative thereof, having the biological activity of a polypeptide encoded by the nucleic acid sequence according to SEQ ID NO:1, into the genome of plant cells or plant tissues and regeneration of the obtained plant cells or plant tissues to plants.

Conventional technologies for producing stable integration of foreign nucleic acid sequences into nucleic acid sequences of host cells are based on electroporation, direct DNA uptake, infectious agents, microinjection, and particle bombardment as described in a number of manuals: Plant Genetic Transformation and Gene Expression: A Laboratory Manual, J. Draper, R. Scott, P. Armitage, R. Walden, 1988, Blackwell Pub; Plant Cell Electroporation and Electrofusion Protocols (Methods in Molecular Biology, 55, J.A. Nickoloff, 1995, Humana Press; Gene Transfer to Plants (Springer Lab Manual), I. Potrykus, G. Spangenberg, 1995, Springer-Verlag Telos; Particle Bombardment for Genetic Engineering of Plants (Biotechnology Intelligence Unit), P. Christou, 1996, R. G. Landes; Genetic Transformation of Plants (Molecular Methods of Plant Analysis New Series), J. F. Jackson, H. F. Linskens, 2003, Springer-Verlag.

The choice of the particular method to be used to transform host cells depends on the species of plant, and these methods are not critical to the current invention. Any appropriate method or combination of methods resulting in the stable integration and expression of the sequence or sequences described in this invention in the desired host cells is acceptable.

For example, as known to a person skilled in the relevant art, the use of *Agrobacterium tumefaciens*-mediated co-transformation using T-DNA binary vectors is the method of choice for many dicotyledonous plants, because it appears to result in high transformation efficiencies and in more predictable segregation patterns of the transgenes when compared to the electroporation or particle delivery systems. With this method single or multiple insertions of the transgene can be obtained that are randomly distributed throughout the nuclear genome.

One aspect of the present invention is a transgenic plant with modified extracellular matrix polymers, e.g. cuticle, having changed diffusivity or strength, thereby displaying an enhanced resistance against e.g. air pollution and pesticides. Said changed diffusivity or strength may result from both increase of the amount of the APB24 protein or RNA and from reduction of the level of the APB24 protein or RNA. Changed diffusivity of the cuticle may be achieved by overexpressing the polypeptide encoded by the nucleic acid sequence according to SEQ ID NO:1 or a homologous nucleic acid sequence thereof in the epidermis of transgenic plants.

Changed diffusivity of extracellular matrix polymers may be regarded as the capacity of preventing water loss through the surface of the plant or as the capacity of reducing speed of chemical diffusion across modified extracellular matrix polymers, e.g. the cuticle. Diffusive properties of the cuticle may be quantified using previously published methods, which estimate the transpiration of water, gas exchange or penetration of radioactively labelled molecules through the cuticle (Niederl *et al*., 1998; Santrucek *et al.,* 2004; Vogg *et al.,* 2004).

Changed strength of extracellular matrix polymers, e.g. the cuticle, may be regarded as the capacity of preventing mechanical, biological or chemical damage of the plant as a whole or fruits and vegetables. In these particular cases, extracellular matrix polymers, e.g. cuticles and epidermal cell walls of transgenic plants obtained according to the present invention, may have more or less α-,ω-dicarboxylic fatty acids that would improve cross-linking in the cell wall and in cuticular polymers. Improved cross-linking should be beneficial to plants by enhancing protective properties of extracellular matrix polymers, e.g. the cuticle and the outermost cell wall of transgenic plants.

Correspondingly, transgenic plants obtained according to the present invention, may have enhanced drought or disease resistance, improved shelf life and transportation behaviour of fruits and vegetables. They also may have reduced scent of flowers, leaves, fruits or roots of the plant. Conversely, reduced cross-linking should result in lessening protective properties of extracellular matrix polymers, e.g. the cuticle and the outermost cell wall of transgenic plants.

Correspondingly, in yet another embodiment, changed strength of the cuticle may be regarded as the capacity of emitting more odorous substances that enhances scent of flowers, leaves, fruits or roots of the transgenic plant. This can be achieved for example by overexpressing the nucleic acid sequence according to SEQ ID NO:1 or a homologous nucleic acid sequence thereof in the epidermis of transgenic plants in the direct (5' to 3') or, preferably, in the opposite (3' to 5') orientation, or, most preferably, in both.

This approach is based on experimental data, known to a person skilled in the art, that the overexpressing nucleic acid may interfere with normal functions of the target nucleic acid through specific hybridisation and result in the phenomena known as co-suppression, transcriptional and posttranscriptional silencing and RNA interference (Cogoni and Macino, 2000; Hammond *et al*., 2001; Schramke and Allshire, 2004). The normal functions of DNA to be interfered with may include replication and transcription. The normal functions of RNA to be interfered with may include splicing of the RNA, translocation of the RNA to the site of protein synthesis and translation of protein from the RNA. As a result of such interference, transgenic plants overexpressing the nucleic acid sequence according to SEQ ID NO:1 or a homologous nucleic acid sequence thereof may not produce or may not produce the sufficient amount of the corresponding polypeptide and, accordingly, may develop the cuticle incapable of reducing the emission of odorous substances that will enhance scent of flowers, leaves, fruits or roots of the transgenic plant.

Another aspect of the present invention is a genetically modified plant having a changed lipid composition.

This may be achieved by overexpressing the polypeptide encoded by the nucleic acid sequence according to SEQ ID NO: 1 or a homologous nucleic acid sequence thereof in the plant organs which are able to accumulate vegetable oil (triacylglycerols) or waxes (esters of very-long-chain (greater than 18 carbons) mono-unsaturated fatty acids) and fatty alcohols, for storage (e.g. seeds). Seeds may be preferably used as they are able to accumulate relatively high amount of oil that can be easily extracted and concentrated, and thus produced at a very low cost.

For this application, native or artificial promoters may be used that allow to express the transgene in the organ or tissue of interest (reviewed by Potenza *et al*., 2004). For example, seed specific expression can be achieved under control of the napin promoter from *Brassica napus* (Broun and Somerville, 1997) or *Arabidopsis thaliana* (Smith et al., 2000) and the hydroxylase promoter from *Lesquerella fendleri* (Broun et al., 1998).

To make use of the very long chain fatty acid ω-alcohol dehydrogenase, which is the subject of this invention, the cells or tissues, in which the said APB24 gene will be overexpressed in transgenic plants, must contain ω-hydroxylated fatty acids that are natural substrates of the enzyme. While these molecules are typically synthesised by the cells producing extracellular matrix polymers, they do not accumulate in the seed oil (the database "Seed oil fatty acids" (SOFA), http://www.bagkf.de/html/sofa.html). Various approaches could be used for engineering of transgenic plants capable of producing ω-hydroxylated fatty acids in the seed oil, and they are not a subject of this invention. One approach could be to express in seeds fatty acid cytochrome P450s monooxygenases, capable of oxidising ω-carbon of fatty acids, such as, for example, fatty acid ω-hydroxylases of the CYP86 and CYP94 gene families (Benveniste *et al.,* 1998; Le Bouquin *et al.,* 1999; Pinot *et al.,* 1999; Wellesen, 2001). Examples that show how seed oil composition can be changed to contain hydroxylated fatty acids have been given (Broun and Somerville, 1997; Smith *et al*., 2000). Also, the use of plant fatty acyl hydroxylases to produce hydroxylated fatty acids and derivatives in plants is a subject of patent applications US 6,028,248 and US 6,433,250.

As a part of the present invention, ω-hydroxylated fatty acids may be specifically oxidised at the ω-carbon through the reaction catalysed by the invented polypeptide *in planta*. Products of this reaction, fatty acids bearing ω-aldehyde, either in a free form or bound, may be extracted from plants, e.g. seeds of the transgenic plants, and refined. The extraction and refinement, e.g. of oil or wax, can be achieved by heat, solvents, or pressure as described by Gunstone and Hamilton (2001).

Also, products of the reaction, catalysed by the invented very long chain fatty ω-alcohol dehydrogenase, may be further oxidised *in planta* to more stable α,ω-dicarboxylic fatty acids by enzymes present in the cells. In principle, such enzymes can be endogenous to plant cells or can be made to be expressed in appropriate host cells or tissues of the plant. For example, enzymes capable of oxidising fatty acids, which bear ω-aldehyde, are CYP94A5, a cytochrome P450 from *Nicotiana tabacum* (Le Bouquin *et al.,* 2001) and the aldehyde dehydrogenase from rabbits (Ichihara *et al.,* 1986a; Ichihara *et al*., 1986b). The products, α,ω-dicarboxylic fatty acids, can be obtained in the plant either in a free form or bound, and could be a part of e.g. oil or wax. Their extraction and refinement are not subjects of the present invention and can be achieved as described by Gunstone and Hamilton (2001).

It is essential in this embodiment that fatty acids obtained from genetically modified plants, tissues or cells get a reactive group, preferably aldehyde or carboxyl group, at the omega side of the aliphatic chain due to activity of the very long chain fatty ω-alcohol dehydrogenase described by this invention. Having reactive groups at both sides of the aliphatic chain adds bifunctionality and allows to use such molecule in cross-linking reactions, in particular, for production of polyesters and monoesters.

The types of reactions and methods of cross-linking are not critical to the current invention. The following patents exemplify the use of aliphatic α,ω-dicarboxylic acids for production of polyesters and monoesters, nylons, fibers, including biodegradable materials, and for the preparation of intermediates for medicines and agricultural chemicals, perfumes, lubricants and adhesives: US 5,962,285; US 6,750,286; US 6,639,045; US 6,417,266; US 6,358,620; US 6,268,465; US 6,350,850; US 6,200,640; US 6,211,317; US 6,355,830; US 6,485,819; US 6,149,995; US 6,750,318; US 6,127,080; US 6,479,618; US 6,468,319; US 6,071,599; US 5,780,582; US 5,962,624; US 6,562,939; US 6,458,858; US 6,120,895; US 5,955,529.

Currently, chemical synthesis of α,ω-dicarboxylic fatty acids is the method of choice, and several chemical routes are available as exemplified by the following patents: US 6,437,180; US 5,973,173; US 5,696,303.

However, most chemical methods result in mixtures of molecules, and extensive purification steps are required, especially, to prepare long-chain α,ω-dicarboxylic fatty acids. Therefore, production of dicarboxylic acids using special microorganisms can be advantageous. The following patents disclose the use of the microbiological approach: US 4,474,882; US 3,975,234; US 4,965,201; US 4,339,536; US 5,962,285; US 5,620,878; GB 1 405 026; DE 2 164 626; DE 2 853 847; DE 2 937 292; DE 2 951 177; DE 2 140 133.

Overexpressing the polypeptide encoded by the nucleic acid sequence according to SEQ ID NO:1 or a homologous nucleic acid sequence thereof in the transgenic plant will provide another way to produce modified fatty acids that carry a reactive group, preferably aldehyde or carboxyl group, at the omega side of the aliphatic chain, based on renewable resources, e.g. seed oil.

Another aspect of the present invention includes the *in vitro* or *in vivo* production of the polypeptide encoded by the nucleic acid sequence according to SEQ ID NO:1 or a homologous nucleic acid sequence thereof.

*In vivo* production can include the use of host cells expressing the nucleic acid sequence according to SEQ ID NO: 1 or a homologous nucleic acid sequence thereof. Various vectors for cloning and expression of a polypeptide in a variety of different host cells currently available (for further details see, for example, Molecular Cloning: a Laboratory Manual: 3rd edition, Sambrook *et al*., 2001, Cold Spring Harbor Laboratory Press) and they are not critical to the current invention.

*In vitro* production can include the use of in vitro translation or transcription/translation systems such as *in vitro* Express™ Translation Kit (Stratagene), TnT Quick Coupled Transcription/Translation System (Promega), Rapid Translation Systems (RTS) from or alike cell-free protein expression systems that comprise an extract prepared from the intracellular contents of cells. These extracts generally contain those molecules which support transcription and protein translation and can be prepared for e.g. from *Escherichia coli* cells, wheat germ cells, rabbit reticulocytes, insect cells and frog oocytes by a person skilled in the art according to manuals (e.g. *In Vitro* Transcription and Translation: 1st edition, M. J. Tymms, 1995, Humana Press).

Another aspect of the present invention includes the use of the polypeptides encoded by the nucleic acid sequence according to SEQ ID NO: or a homologous nucleic acid sequence thereof for enzymatic reactions with appropriate substrates or in assays. This includes using the polypeptides encoded by the nucleic acid sequence according to SEQ ID NO: 1 or a homologous nucleic acid sequence thereof in screening assays to identify inhibitors.

The ω-hydroxyfatty acid dehydrogenase encoded by the nucleic acid sequence according to SEQ ID NO: 1 or a homologous nucleic acid sequence thereof converts long-chain ω-hydroxy fatty acids, which are natural substrates *in planta*, to fatty aldehydes. For enzymatic reactions, the said polypeptide expressed in host cells or cell-free protein expression systems can be incubated in a reaction buffer with a chemical compound which is not necessarily a natural substrate. For example, any organic compound carrying a hydroxyl group that can be oxidised to aldehyde group due to enzymatic activity of the polypeptides described herein can be used for enzymatic reactions in vitro or in assays. Organic compounds may be characterised by the presence of carbon-hydrogen bonds, saturated or unsaturated chains of carbon, aromatic hydrocarbons and various functional groups. Millions of such compounds are known that constitute the study of organic chemistry (for further details see, for example, Organic Chemistry: 5th Edition, L. G. Wade, L. G., Jr. Wade, 2002, Prentice Hall) and they are not critical to the current invention.

It is a part of this embodiment, that enzymatic reactions or assays with the polypeptides encoded by the nucleic acid sequence according to SEQ ID NO: or a homologous nucleic acid sequence thereof, or cells expressing the said polypeptides are used for testing which chemical compounds are capable of acting as enzyme substrates.

For enzymatic reactions *in vitro*, the reaction buffer may include various components such as buffering components, salts, cofactors (e.g. nicotinamide adenine dinucleotide (NAD)), stabilising agents (e.g. inert proteins), microsomal membranes, which are added to keep the enzyme in the active state, to facilitate enzymatic reactions. Various compositions of the reaction buffer are possible, and this is not critical to the current invention. An example of the reaction buffer for plant ω-hydroxyfatty acid dehydrogenase has been provided (Agrawal and Kolattukudy, 1978a; Agrawal and Kolattukudy, 1978b).

It is a part of this embodiment, that host cells expressing the polypeptides encoded by the nucleic acid sequence according to SEQ ID NO:1 or a homologous nucleic acid sequence thereof can be used for testing which chemical compounds are capable of acting as enzyme substrates.

In addition, the above assay may include various chemical inhibitors, preferably organic compounds. It is a part of this embodiment, that enzymatic reactions or assays with the polypeptides encoded by the nucleic acid sequence according to SEQ ID NO:1 or a homologous nucleic acid sequence thereof are used for testing which chemical compounds are capable of acting as enzyme inhibitors.

It is a part of this embodiment, that host cells expressing the polypeptides encoded by the nucleic acid sequence according to SEQ ID NO: 1 or a homologous nucleic acid sequence thereof can be used for testing which chemical compounds are capable of acting as enzyme inhibitors.

The course of enzymatic reactions *in vitro* or in the host cells can be followed using various approaches, direct and indirect methods and technologies that are not critical to the current invention (for further details see, for example, High Throughput Screening: Methods and Protocols (Methods in Molecular Biology, 190), W. P. Janzen, 2002, Humana Press; Integrated Drug Discovery Technologies, Houng-Yau Mei, A. W. Czarnik, 2002, Marcel Dekker; High Throughput Screening: The Discovery of Bioactive Substances, J. P. Devlin, 1997, Marcel Dekker).

For example, as it is known to a skilled person in the art, in order to identify inhibitors it is possible to incubate an enzymatic reaction mix or a cellular constituent, e.g. microsomal membrane, or host cells containing the polypeptides to be used according to the invention, together with a labelled substrate in the presence and absence of a candidate chemical compound, which may be capable of acting as enzyme inhibitor. The inhibitory ability of the candidate chemical compound may evident from an increased or reduced conversion of the labelled substrate.

In the other example, the activity of the enzyme in the presence and absence of a candidate chemical compound, which may be capable of acting as enzyme inhibitor, can be determined by a spectrophotometric assay for conversion of the cofactor present in the enzymatic reaction. In particular, as it is known to a skilled person in the art, the increase in absorbance at 340 nm, caused by the reduction of NADP⁺ to NADPH is a measure of the catalytic activity of many dehydrogenases.

In the other example, the activity of the enzyme in the presence and absence of a candidate chemical compound, which may be capable of acting as enzyme inhibitor, can be evident from the amount of product accumulated in the enzymatic reaction. In particular, as it is known to a skilled person in the art, ω-oxofatty acids, which are the natural products of enzymatic activity of the polypeptides to be used according to the invention, can be derivatised with 2,4-dinitrophenyl-hydrazine (DNPH), which forms derivatives with aldehydes and ketones. The increase in absorbance at 360-365 nm provides a measure of the formation of oxofatty acids (for further experimental details see, for example Weichert H. *et al*., FEBS Letters 464 (1999) 133-137), and allows to estimate catalytic activity of the enzyme and inhibitory effect of a candidate chemical compound.

In the other example, candidate inhibitory compounds can be identified by the screening procedures that are based rather on protein-ligand interaction than on enzymatic assays. The binding of chemical compounds, which are relatively small molecules, to the polypeptides to be used according to the invention, can be evaluated using a number of various approaches and methods, which are known to a skilled person in the art. The detection can be based on but not limited to surface plasmon resonance (SPR) recording, fluorescence correlation spectroscopy and surface-enhanced laser desorption/ionisation analysis (for further details see, for example, Single-Molecule Detection in Solution Methods and Applications, C. Zander, J. Enderlein, R. A. Keller (Eds), 2002, Wiley-VCH; Fluorescence Correlation Spectroscopy, R. Rigler, R. Rigler, E. S. Elson, 2001, Springer-Verlag; Quantitative Analysis of Biospecific Interactions, P. Lundahl, A. Lundqvist, E. Greijer, 1998, Gordon & Breach Science Publishers).

It is a part of this embodiment, that chemical inhibitors identified as described above, may be herbicidal compounds that can be used to kill or suppress the growth of plant cells, tissues or a whole plant.

With regard to the protection of the plant body against water loss, pathogens, solar radiation, pollutants and industrial chemicals extracellular matrix polymers are absolutely essential (reviewed in Kolattukudy, 2001a; Kolattukudy, 2001b; Kolattukudy, 2001c). Therefore, the suppression of ω-hydroxyfatty acid dehydrogenase enzymatic activity of the polypeptides to be used according to the invention, can result in the cell death or the death of the plant, or suppression of growth and development. With this respect, the said polypeptides can be potential herbicide targets.

Therefore, another aspect of the present invention includes the use of the polypeptides encoded by the nucleic acid sequence according to SEQ ID NO: or a homologous nucleic acid sequence thereof in the screening for chemical inhibitors as described above when these chemical inhibitors are to be used as herbicides. Once an inhibitor has been identified, the next step can be to apply it to plants at various concentrations. After application of the inhibitor, preferably by spraying, its herbicidal effects on plants can be recorded, including cell death, and suppression of growth and development.

Another aspect of the present invention resides in the use of an inhibitory acting compound preferably identified by a method as described above for controlling undesirable vegetation. Such use comprises the application of at least one compound having an inhibitory effect on fatty acid ω-alcohol dehydrogenase to said undesirable plants and/or their seeds. The inhibitor may be applied not only to said plants or seeds, but also to the areas where said plants grow.

### Example 1: Cuticular defects associated with the loss of the APB24 function (figure 1)

The transposon-induced mutant APB24 of Arabidopsis shows severe cuticular defects, upon inspection of the epidermis by transmission electron microscopy. For transmission electron microscopy, plant materials were fixed with a mixture of glutaraldehyde (2%) and formaldehyde (4%) in sodium cacodylate (0.05 M, pH 7.2) for 2 hr and postfixed with OsO₄ (1%) in sodium cacodylate at 4°C overnight, dehydrated with ethanol and embedded in LR White resin (London Resin Company Ltd., UK) at 60°C for 24 h. Ultrathin sections (50-100 nm) were double-stained with uranyl acetate (5%) for 30 min and Reynolds lead citrate for 2 min, and then examined with a Zeiss EM 10C microscope.
Cuticular defects associated with the loss of the APB24 function are evident by comparing Fig 1A and Fig. 1B, 1C. The dark layer above the light cell wall (cuticle proper) is continuous in a wild type plant (Fig. 1A) and discontinuous (Fig. 1B) or multilayered (Fig. 1C) in the APB24 mutant. In the mutant, the direct contact of cell wall material is possible, therefore cell wall fusions between distinct organs occur as illustrated in the Fig. 1C.
This example shows that activity of the very long chain fatty ω-alcohol dehydrogenase described by this invention is required for the formation of the cuticle proper at the outermost cell walls of the epidermis.

### Example 2: Metabolic analysis (figure 2)

With the use of GC-MS, fatty acid metabolites were compared in the APB24 mutants and wild type plants.
In the first experiment, lipids were isolated from floral buds as described previously (Browse *et al*., 1986) with modifications. Briefly, 30 mg of lyophilised tissues were incubated in closed glass tubes with 1 ml 1 N methanolic-HCl (Supelco) and 5% (v/v) 2,2-dimethoxypropan. Before incubation for 1 h at 80°C, the tubes have been flashed with argon. The reaction mixture was then cooled to room temperature and extracted with shaking for 10 min with 0.3 ml heptane in the presence of 1 ml 0.9% NaCl. The water and organic phases were separated by centrifugation at 800 rpm for 5 min. The heptane phase was transferred into a GC microvial and analysed with a Hewlett Packard 5890 series II gas chromatograph coupled to the HP 5973 Mass Selective Detector. An HP-5MS methyl phenyl silicone capillary column (10 m x 0.20 mm) was used.
Chromatographic parameters were: injector temperature 250°C; initial oven temperature 150°C for 1 min; the temperature was then raised to 320°C at 12°C/min, and held there for 5 min. Peak areas were corrected before comparison with the area of the α-linolenic acid peak, which was used instead of an internal standard.
With this method, under conditions known to cleave ester bonds, extraction and transesterification of total fatty acids occurred in one step. Free fatty acids and fatty acids linked by ester bonds e.g. to glycerol, aliphatic and aromatic alcohols, extracellular matrix polymers, including cutin and suberin, were extracted into organic phase for analysis.
Fatty acid metabolites were identified by their retention times and their mass spectra, and the ratios of peak areas in APB24 and wild type were compared (Fig. 2A). While ratios close to 1 were obtained for most of the compounds, the ratios for five metabolites were altered by up to two-fold (Fig. 2B). To establish the molecular identities of these metabolites, their mass spectra were compared to those of reference substances in mass spectra libraries. One compound showed a marked increase in the APB24 mutant, and was identified as 18-hydroxyoctadecadienoic acid (peak 3), while ω-oxo fatty acids with aliphatic chains of 16 and 18 carbons in length (peaks 2 and 5, respectively) were present in reduced amounts. The other compounds whose levels were lower in the mutant were the end products of ω-oxidation - C16 and C18 α-,ω-dicarboxylic acids (peaks 1 and 4, respectively). These results clearly indicate that the APB24 mutant is defective in the oxidation of long-chain ω-hydroxy fatty acids to ω-oxo fatty acids.

In the second experiment, the insoluble polymeric material isolated from leaves of the APB24 mutant and wild type plants was analysed.
For this analysis, leaves from wild type and mutant plants were thoroughly steeped in organic solvents to extract all soluble lipids. The remaining polyester lipids were subjected to hydrolysis and analysed by GC-MS.
Briefly, wild types and progeny of heterozygous plants were grown until flowering to identify APB24 mutants. Mature leaves (about 300 mg per sample) were harvested and their areas measured by scanning. Soluble lipids were extracted from samples by steeping in 25 ml of methanol/chloroform (1:1) mixture for 7 days, changing the solvent daily. After drying, the leaf material was weighed (typically about 30 mg) and stored or used directly for analysis.
Transesterification of the residual bound lipids was performed with 6 ml of 1 N HCl in methanol (2 hr, 80°C) in a Teflon-sealed screw-cap tube. The hydrophobic monomers were subsequently extracted into hexane (three times with 6 ml) containing 20 µg of dotriacontane (C32) as an internal standard. After evaporating the solvent to 100 µl at 50°C under a stream of N₂, free hydroxyl groups were converted into their trimethylsilyl ethers with 20 µl of *N*,*N-*bis-trimethylsilyltrifluoroacetamide (BSTFA) and 20 µl of pyridine (40 min, 70°C). Samples were analysed with GC-MS using a gas chromatograph 6890N (DB-1.30 m x 0.32 mm, 0.1 µm (J&W) equipped with a quadropole mass selective detector 5973N (Agilent Technologies, Böblingen, Germany), Quantitative determination of lipids was carried out with the same GC system equipped with a flame ionisation detector (FID).
With this method, only residual bound lipids of extracellular matrix polymers, were analysed. After cleaving ester bonds and transesterification with methanolic HCl similar to that described above in the first experiment, free hydroxyl groups were derivatised by silylation with BSTFA to optimise separation of metabolites and enable their identification.
Many of the fatty acid derivatives liberated from non-solubilisable leaf lipids were constituents commonly found in both cutin and suberin, but di-unsaturated C18 fatty acids were also detected (Fig. 2C). No ω-oxo fatty acids were found in the non-solubilisable leaf lipids, suggesting that they are converted into α-,ω-dicarboxylic fatty acids before incorporation into polyesters. Lower than normal levels of α-,ω-dicarboxylic fatty acids, and elevated levels of ω-hydroxy fatty acids, were identified in the leaf polyesters isolated from the APB24 mutant (Fig. 2D).
This example shows that the loss of activity of the very long chain fatty ω-alcohol dehydrogenase described by this invention results in the reduction in the levels of α-,ω-dicarboxylic fatty acids in the insoluble polymeric material isolated from the APB24 mutant.

Therefore, the deformation of the cuticle proper at the outermost cell walls of the epidermis must be attributed to the inability to cross-link extracellular lipids in the cell wall or/and in the cuticle proper.
In the third experiment, composition of residual bound lipids and cutin from *B. oleracea* were compared. The aim of this experiment was to find out whether composition of residual bound lipids, analysed as described in the second experiment, and composition of cutin are different. In Arabidopsis, isolated cutin often contains cell-wall contaminants, and it is difficult to define which α-,ω-dicarboxylic fatty acids are cell wall constituents and which are derived from cutin monomers. We therefore compared the chemical composition of cutin with that of the insoluble polymeric material in leaves of a close relative of Arabidopsis, *Brassica oleracea* var. *italica* (broccoli), in which isolation of cutin is feasible.
For this analysis, plants were grown in the open, and leaf discs (18 mm diameter) were punched out with a cork borer using freshly harvested leaf material. Cuticular membranes were enzymatically isolated (based on J. Schoenherr, M. Riederer, Plant Cell Environ. 9, 459-466 (1986)) from adaxial leaf surfaces by incubating in a mixture (1:1) of cellulase (Celluclast, Novo Nordisk, Bagsvaerd, Denmark) and pectinase (Trenolin, Erbslöh, Geisenheim, Germany) at a concentration of 1% (w/w). After a few days, cuticles were collected and air-dried. To prepare polymer matrix membranes (cutin), cuticle disks were cleared in chloroform, which extracts soluble lipids e.g. cuticular waxes. Samples for analysis of residual bound lipids were obtained from leaf disks by steeping as described in the second experiment.
Polymer matrix membranes (cutin) and the insoluble polymeric material isolated from leaf disks were analysed with GC-MS as described in the second experiment of this example.
This experiment revealed that saturated α-,ω-dicarboxylic fatty acids in the insoluble polymeric material are derived from cutin, while unsaturated α-,ω-dicarboxylic fatty acids are not (Fig. 2E). It follows, therefore, that the very long chain fatty ω-alcohol dehydrogenase described by this invention is involved in the biosynthesis of both cell-wall bound and cutin lipids, the composition of which is not necessarily identical to each other.

### Example 3: Expression in the epidermis

The aim of these experiments was to study whether the APB24 gene specifically acts in dermal cells.
In the first experiment, mRNA *in situ* hybridisation was performed with a probe to the APB24 gene.

*In situ* hybridisation was performed mainly as previously described (Huijser *et al*., 1992) with modifications. Tissues were immersed in 100 mM phosphate buffer, pH 7.2 (prepared as 68.4 parts of Na₂HPO₄ plus 31.6 parts of NaH₂PO₄) containing 4% para-formaldehyde for fixation and evacuated several times for 15 min. until the tissue sinks. To remove fixative the tissue was rinsed in 3×15 min in the 100 mM phosphate buffer. After dehydration in series of ethanol (EtOH) solutions from 30 to 70% the tissue was incubated overnight or longer in 70% EtOH until complete destaining. Dehydration was completed in 85%, 95% and 100% EtOH for 1 hour each.
Clearing was performed in series of EtOH:Histoclear mixtures (2:1,1:1,1:2) for 30 min each time and in 100% Hystoclear for 4 hours at RT. Infiltration with paraffin (Paraplast Plus® from Oxford Labware) was conducted in an vacuum oven at 60°C. 6 changes of Hystoclear:paraffin (1:5) were made over 3 days.
Before sectioning the samples were evacuated and rapidly cooled in ice-cold water. Blocks of paraffin embedded tissue were cut to 7-8 µm sections on a microtome, placed on glass slides and dried on a slide warmer at 42°C for 24-48 hours.
The digoxigenin-labelled RNA probe was synthesised from a 1600-nt PCR product prepared from a cDNA by PCR with the ACE-SP6 (CTCGAGTTTAGGTGACACTATAGAACTGGAGCCTCCACTGCCTCTAAAGGTAAA GAG) (SEQ ID NO:4) and ACE-T7 (CTCGAGTAATACGACTCACTATAGGGAGACACCAAGAACTTTGCGGTTAGGG) (SEQ ID NO:5) primers (the SP6 and T7 promoters are underlined) using DIG RNA Labelling Mix (Boehringer Mannheim) according to provided protocol.
Paraffin was removed from sections before hybridisation in series of 5 min. incubations in HystoClear:EtOH mixtures (3×1:0, 1× 1:1, 1× 0:1) in a staining slide holder. The sections were rehydrated by dipping them in EtOH solutions prepared with 0.85% NaCl (sequentially in 95%, 85%, 70%, 50% and 30% EtOH) and finally in 0.85% NaCl for 2 min each time. Denaturation with 0.2N HCl, Proteinase K digestion and dehydration were performed as described (Huijser *et al*., 1992).
Sections covered with hybridisation solution and coverslips were incubated overnight at 50°C in a humid chamber. Posthybridisation washing and RNAse treatment was done as described (Huijser *et al*., 1992). Immunological detection was performed using anti-DIG AP conjugates (Boehringer Mannheim) according to manufacturer's instructions. Staining reaction with Nitro Blue Tetrazolium chloride (NTB) and 5-Bromo-4-chloro-3-indolyl,4-toluidine salt (BCIP) was completed after 12-14 hour incubation. Cell walls were optionally stained for 20 min with Calcofluor® (Sigma). Slides were dried and mounted for storage using Entellan (Merck). Sections were viewed under fluorescent light or under bright-field illumination with a Zeiss Axiophot microscope.
As it is evident from Fig. 8I-8L, the expression of the APB24 gene was found to be specific to the protoderm and epidermis in all organs studied.

In the second experiment, the promoter of the APB24 gene has been localised to the 5' upstream region.
The 1.9-kb promoter of the APB24 gene was obtained by PCR with the primers ACE-Hind (AAAAAAAGCTTCAAAAGAGGACTAAAGAGGAGAGAAAC) (SEQ ID NO:6) and ACE-Xba (AAAAAATCTAGACTTATGTGGCTTTTGATGAATGGTC) (SEQ ID NO:7), which introduced *Hin*dIII and *Xba*I cloning sites (underlined), respectively, at the 5' and 3' ends of the promoter.
Two APB24 promoter-reporter binary vectors containing the β-glucuronidase (GUS) or green fluorescence protein (GFP) mgfp5-ER (Haseloff and Siemering, 1998) reporter gene, were constructed in the binary vector pBHS (Wellesen *et al*., 2001).

Transgenic plants of Arabidopsis were obtained as described (Bechtold *et al.,* 1993) by vacuum infiltration with *Agrobacterium tumefaciens* GV3101 harbouring pMP90^{Gent} disarmed Ti-plasmid and binary vectors. Recombinant *A. tumefaciens* cultures were maintained at 100 µg/ml rifampicin, 25 µg/ml gentamicin and 25 µg/ml kanamycin. Transgenic plants were selected by spraying with a BASTA (Hoechst) herbicide solution (0.1 % for one-two week old plants, 0.5% for two-three week old plants and 1% for four week old plants).
For confocal laser scanning microscopy of tissues expressing GFP under the control of the APB24 promoter, GFP fluorescence in transgenic plants was examined with a Leica TCS 4D confocal laser scanning microscope using a fluorescein isothiocyanate filter set. For analysis, fresh tissues were mounted in 5% Small DNA Low Melt Agarose (Biozym, Hessisch-Oldendorf, Germany) containing 0.005% Silwet L-77 (OSi Specialities, Slough, UK). Agarose blocks of about 0.5 x 0.5 x 1 cm were mounted on a Leitz 1512 Vibratome stage and sections of 100-150 µm were cut.
For histochemical expression analysis, portions of tissue or whole seedlings of transgenic plants, bearing APB24 promoter-GUS fusions, were stained for GUS activity according to Jefferson, *et al*., 1987 except that K₃Fe(CN)₆ and K₄Fe(CN)₆ were omitted from the staining solution. To detect GUS expression, plants were stained in 0.75 mg/ml 5-bromo-4-chloro-3-indoly-β-D-glucuronid acid, 0.1 M sodium phosphate buffer, pH 7.0, 20% methanol. Before examination, plant tissue was cleared in 70% ethanol.

As Fig. 8A-8F show, the expression of the very long chain fatty ω-alcohol dehydrogenase described by this invention could be localised to the protodermis and epidermis, confirming results of mRNA *in situ* hybridisation obtained in the first experiment of this example. It follows, that the said gene has the specific function in oxidation of ω-hydroxyfatty acids, which are required for biosynthesis of extracellular matrix polymers in the epidermis. As shown by Fig. 8G and 8H, the activity of the said gene could be detected in accord with its function in the cells, which had to rapidly produce extracellular matrix polymers.
Furthermore, transcriptional regulatory sequences required for the epidermis-specific expression of the APB24 gene, encoding the very long chain fatty ω-alcohol dehydrogenase described by this invention, are located within the fragment as defined in SEQ ID NO:3.

### References

Agrawal, V. P., and Kolattukudy, P. E. (1978a). Mechanism of action of a wound-induced ω-hydroxyfatty acid, NADP oxidoreductase from potato tuber Solanum tuberosum L. Archives Biochem Biophys *191*, 466-478.
Agrawal, V. P., and Kolattukudy, P. E. (1978b). Purification and characterization of a wound-induced ω-hydroxy fatty acid: NADP: oxidoreductase from potato tuber disks (Solanum tuberosum L.). Archives Biochem Biophys *191*, 452-465.
Araki, T., Nakatani, M., and Iwabuchi, M. (1998). ADHESION OF CALYX EDGES, a gene involved in the regulation of postgenital fusion in Arabidopsis. Paper presented at: 9th International Conference on Arabidopsis Research (University of Wisconsin, Madison).
Araki, T., and Nakatani-Goto, M. (1999). Arabidopsis ADHESION OF CALYX EDGES (ACE), genomic. Published Only in DataBase.
Bechtold, N., Ellis, J., and Pelletier, G. (1993). In planta *Agrobacterium* gene transfer by infiltration of adult *Arabidopsis thaliana* plants. C R Acad Sci Paris, Life Sciences *316,* 1194-1199.
Benveniste, I., Tijet, N., Adas, F., Philipps, G., Salaun, J.-P., and Durst, F. (1998). CYP86A1 from Arabidopsis thaliana encodes a cytochrome P450-dependent fatty acid omega-hydroxylase. Biochem. Biophys. Res. Comm. 243, 688-693.
Bernards, M. A. (2002). Demystifying suberin. Canad. J. Bot. 80, 227-240.
Bremer, J., and Osmundsen, H. (1984). Fatty acid oxidation and its regulation. In Fatty Acid Metabolism and Its Regulation, S. Numa, ed. (Amsterdam, Elsevier Science Publishers B.V.), pp. 113-154.
Broun, P., Boddupalli, S., and Somerville, C. (1998). A bifunctional oleate 12-hydroxylase: desaturase from *Lesquerella fendleri*. Plant J *13*, 201-210.
Broun, P., and Somerville, C. (1997). Accumulation of ricinoleic, lesquerolic, and densipolic acids in seeds of transgenic Arabidopsis plants that express a fatty acyl hydroxylase cDNA from castor bean. Plant Physiol *113*, 933-942.
Browse, J., McCourt, P. J., and Somerville, C. R. (1986). Fatty acid composition of leaf lipids determined after combined digestion and fatty acid methyl ester formation from fresh tissue. Anal Biochem *152*, 141-145.
Cavener, D. R. (1992). GMC oxidoreductases. A newly defined family of homologous proteins with diverse catalytic activities. J. Mol. Biol. 223, 811-814.
Cogoni, C., and Macino, G. (2000). Post-transcriptional gene silencing across kingdoms. Curr Opin Genet Dev *10*, 638-643.
Fink, G., Montgomery, J., David, F., Gameau, M., Livni, E., Elmaleh, D., Strauss, H., and Brunengraber, H. (1990). Metabolism of beta-methyl-heptadecanoic acid in the perfused rat heart and liver. J Nucl Med *31*, 1823-1830.
Gunstone, F. D., and Hamilton, R. J. (2001). Oleochemical Manufacture & Applications (CRC Press)
Hammond, S. M., Caudy, A. A., and Hannon, G. J. (2001). Post-transcriptional gene silencing by double-stranded RNA. Nat Rev Genet 2, 110-119.
Haseloff, J., and Siemering, K. R. (1998). The uses of green fluorescent protein in plants. In Green fluorescent protein: Properties, applications, and protocols., M. Chalfie, and S. Kain, eds. (Chichester, John Wiley and Sons Ltd. ; Wiley-Liss, Ltd. , Chichester, England), pp. 191-220.
Holloway, P. J. (1982). The chemical constitution of plant cutins. In The Plant cuticle : papers presented at an international symposium organized by the Linnean Society of London, held at Burlington House, London, 8-11 September 1980, D. F. Cutler, K. L. Alvin, and C. E. Price, eds. (London, Academic Press), pp. 45-85.
Holloway, P. J. (1983). Some variations in the composition of suberin from the cork layers of higher plants. Phytochemistry 22, 495-502.
Huijser, P., Klein, J., Loennig, W. E., Meijer, H., Saedler, H., and Sommer, H. (1992). Bracteomania: an inflorescence anomaly is caused by the loss of function of the MADS-box gene *SQUAMOSA* in *Antirrhinum majus.* EMBO J *11*, 1239-1249.
Ichihara, K., Kusunose, E., Noda, Y., and Kusunose, M. (1986a). Some properties of the fatty alcohol oxidation system and reconstitution of microsomal oxidation activity in intestinal mucosa. Biochimica et Biophysica Acta (BBA) - Lipids and Lipid Metabolism 878, 412-418.
Ichihara, K., Noda, Y., Tanaka, C., and Kusunose, M. (1986b). Purification of aldehyde dehydrogenase reconstitutively active in fatty alcohol oxidation from rabbit intestinal microsomes. Biochimica Et Biophysica Acta 878, 419-425.
Jefferson, R. A., Kavanagh, T. A., and Bevan, M. W. (1987). *GUS* fusions: β-glucuronidase as a sensitive and versatile gene fusion marker in higher plants. EMBO J 6, 3901-3908.
Krolikowski, K. A., Victor, J. L., Wagler, T. N., Lolle, S. J., and Pruitt, R. E. (2003). Isolation and characterization of the Arabidopsis organ fusion gene *HOTHEAD.* Plant J 35, 501-511.
Le Bouquin, R., Pinot, F., Benveniste, I., Salaun, J.-P., and Durst, F. (1999). Cloning and functional characterization of CYP94A2, a medium chain fatty acid hydroxylase from *Vicia sativa.* Biochem Biophys Res Comm 261, 156-162.
Le Bouquin, R., Skrabs, M., Kahn, R., Benveniste, I., Salaun, J.-P., Schreiber, L., Durst, F., and Pinot, F. (2001). CYP94A5, a new cytochrome P450 from *Nicotiana tabacum* is able to catalyze the oxidation of fatty acids to the ω-alcohol and to the corresponding diacid. Eur J Biochem 268, 3083-3090.
Lee, T. (1979). Characterization of fatty alcohol:NAD+ oxidoreductase from rat liver. The Journal Of Biological Chemistry 254, 2892-2896.
Marchler-Bauer, A., Anderson, J. B., DeWeese-Scott, C., Fedorova, N. D., Geer, L. Y., He, S., Hurwitz, D. I., Jackson, J. D., Jacobs, A. R., Lanczycki, C. J., *et al*. (2003). CDD: a curated Entrez database of conserved domain alignments. Nucl Acids Res 31, 383-387.
Matzke, K., and Riederer, M. (1991). A comparative study into the chemical constitution of cutins and suberins from Picea abies (L.) Karst., Quercus robur L., and Fagus sylvatica L. Planta 185, 233-245.
Nelson, D. R., Koymans, L., Kamataki, T., Stegeman, J. J., Feyereisen, R., Waxman, D. J., Waterman, M. R., Gotoh, O., J., C. M., W., E. R., *et al*. (1996). P450 superfamily: Update on new sequences, gene mapping, accession numbers and nomenclature. Pharmacogenetics *6*, 1-42.
Niederl, S., Kirsch, T., Riederer, M., and Schreiber, L. (1998). Co-permeability of ³H-labeled water and ¹⁴C-labeled organic acids across isolated plant cuticles . Investigating cuticular paths of diffusion and predicting cuticular transpiration. Plant Physiol *116*, 117-123.
Pinot, F., Benveniste, I., Salaun, J.-P., Loreau, O., Noel, J.-P., Schreiber, L., and Durst, F. (1999). Production in vitro by the cytochrome P450 CYP94A1 of major C18 cutin monomers and potential messengers in plant-pathogen interactions: Enantioselectivity studies. Biochem J *342*, 27-32.
Potenza, C., Aleman, L., and Sengupta-Gopalan, C. (2004). Invited review: Targeting transgene expression in research, agricultural, and environmental applications: Promoters used in plant transformation. In Vitro Cell Dev Biol-Plant *40*, 1-22.
Rizzo, W. B., Craft, D. A., Dammann, A. L., and Phillips, M. W. (1987). Fatty alcohol metabolism in cultured human fibroblasts. Evidence for a fatty alcohol cycle. J. Biol. Chem. *262*, 17412-17419.
Santrucek, J., Simanova, E., Karbulkova, J., Simkova, M., and Schreiber, L. (2004). A new technique for measurement of water permeability of stomatous cuticular membranes isolated from Hedera helix leaves. J Exp Bot 55, 1411-1422.
Schramke, V., and Allshire, R. (2004). Those interfering little RNAs! Silencing and eliminating chromatin. Curr Opin Genet Dev *14*, 174-180.
Smith, M., Moon, H., and Kunst, L. (2000). Production of hydroxy fatty acids in the seeds of Arabidopsis thaliana. Biochem Soc Trans 28, 947-950.
Sumimoto, H., and Minakami, S. (1990). Oxidation of 20-hydroxyleukotriene B4 to 20-carboxyleukotriene B4 by human neutrophil microsomes. Role of aldehyde dehydrogenase and leukotriene B4 omega-hydroxylase (cytochrome P-450LTB omega) in leukotriene B4 omega-oxidation. J Biol Chem *265*, 4348-4353.
Vamecq, J., De Hoffmann, E., and Van Hoof, F. (1985). The microsomal dicarboxylyl coenzyme A. Biochem. J. *230,* 683-694.
Vanhanen, S., West, M., Kroon, J. T. M., Lindner, N. 1., Casey, J., Cheng, Q., Elborough, K. M., and Slabas, A. R. (2000). A consensus sequence for long-chain fatty-acid alcohol oxidases from Candida identifies a family of genes involved in lipid omega-oxidation in yeast with homologues in plants and bacteria. J.Biol. Chem. *275,* 4445-4452.
Vogg, G., Fischer, S., Leide, J., Emmanuel, E., Jetter, R., Levy, A. A., and Riederer, M. (2004). Tomato fruit cuticular waxes and their effects on transpiration barrier properties: functional characterization of a mutant deficient in a very-long-chain fatty acid β-ketoacyl-CoA synthase. J Exp Bot *55*, 1401-1410.
Wellesen, K., Durst, F., Pinot, F., Benveniste, I., Nettesheim, K., Wisman, E., Steiner-Lange, S., Saedler, H., and Yephremov, A. (2001). Functional analysis of the *LACERATA* gene of Arabidopsis provides evidence for different roles of fatty acid omega-hydroxylation in development. Proc Natl Acadl Scil USA 98, 9694-9699.
Werck-Reichhart, D., Bak, S., and Paquette, S., eds. (2001). Cytochromes P450 (http://www.aspb.org/publications/arabidopsis).
Wheelan, P., Hankin, J. A., Bilir, B., Guenette, D., and Murphy, R. C. (1999). Metabolic Transformations of Leukotriene B4 in Primary Cultures of Human Hepatocytes. J Pharmacol Exp Ther 288, 326-334.

## Claims

1. A method for the generation of a transgenic plant having a changed lipid composition and/or modified extracellular matrix polymers, the method comprising the step of stably integrating at least one deoxyribonucleic acid having the nucleotide sequence according to SEQ ID NO:1, or a fragment thereof, or of a nucleic acid hybridising to the nucleic sequence according to SEQ ID NO:1 under stringent conditions, wherein said at least one deoxyribonucleic acid or said fragment thereof, or said nucleic acid hybridising to the nucleic sequence, is expressed in antisense or sense-orientation in respect to the endogenous sequence responsible for the changed lipid composition, into the genome of a plant cell, plant tissue, protoplast or plant propagation material by transformation of said plant cell, plant tissue, protoplast or plant propagation material and regeneration of the obtained plant cell or plant tissue to a transgenic plant.

2. The method according to claim 1, wherein the expression of said at least one deoxyribonucleic acid having the nucleotide sequence according to SEQ ID NO:1, or said fragment thereof, or said nucleic acid hybridising to the nucleic sequence according to SEQ ID NO:1 under stringent conditions is controlled by a tissue or organ specific promoter.

3. The method according to claim 2, wherein the promoter is selected from the napin promoter from *Brassica napus* or from *Arabidopsis thaliana,* or the hydroxylase promoter from *Lesquerella fendleri*.

4. The method according to any one of claims 1 to 3, wherein the plant cell, plant tissue, protoplast or plant propagation material is derived from a monocotyledonous or dicotyledonous plant, such as rapeseed, canola, tomato, potato, safflower, sunflower, soybean, peanut, cotton, palm, maize, sorghum, rice, barley and wheat.

5. A transgenic plant, obtainable by the method according to any one of claims 1 to 4.

6. A transgenic plant, plant cell, plant tissue, protoplast or plant propagation material transformed with an expressible deoxyribonucleic acid having the nucleotide sequence according to SEQ ID NO:1, or a fragment thereof, or a nucleic acid hybridising to the nucleic sequence according to SEQ ID NO:1 under stringent conditions, wherein the expressible deoxyribonucleic acid is stably integrated into the genome of the plant, plant cell, plant tissue, protoplast or plant propagation material.

7. The transgenic plant, plant cell, plant tissue, protoplast or plant propagation material according to claim 6, which is regenerable to a seed forming plant.

8. A seed or a fruit transgenic in the nucleic acid having the nucleotide sequence according to SEQ ID NO:1, or a fragment thereof, or a nucleic acid hybridising to the nucleic sequence according to SEQ ID NO:1 under stringent conditions, obtainable from a plant according to any of claims 5 to 7.

9. Use of a transgenic plant according to any of claims 5 to 7 for the production of α-,ω-dicarboxylic fatty acids, such as fatty acids bearing an omega-aldehyde or omega-carboxyl group.

10. A method of identifying an inhibitor of a polypeptide encoded by the nucleic acid sequence according to SEQ ID NO:1 or a homologous nucleic acid sequence thereof, the method comprising the following steps:
(a) contacting a host cell, said host cell including a polypeptide, said polypeptide having the biological activity of a fatty acid ω-alcohol dehydrogenase and comprising an amino acid sequence having at least 80% identity with the sequence according to SEQ ID NO:2, with a chemical compound or a mixture of chemical compounds under conditions which permit the interaction of the chemical compound or the mixture of chemical compounds with the polypeptide,
(b) comparing the biological activity of the polypeptide in the presence of the chemical compound or the mixture of chemical compounds with the biological activity of the polypeptide in the absence of the chemical compound or the mixture of chemical compounds, or
(c) determining binding of the chemical compound or the mixture of chemical compounds to the polypeptide and
(d) determining the chemical compound which specifically modulates the biological activity of the polypeptide or binds to the polypeptide, thereby identifying the inhibitor.

11. The method according to claim 10, wherein said polypeptide comprises the amino acid sequence of SEQ ID NO:2.

12. A method of identifying an inhibitor of a polypeptide encoded by the nucleic acid sequence according to SEQ ID NO:1 or a homologous nucleic acid sequence thereof, the method comprising the following steps:
(a) contacting a polypeptide having the biological activity of fatty acid ω-alcohol dehydrogenase and comprising an amino acid sequence having at least 80% identity with the sequence according to SEQ ID NO:2, with a chemical compound or a mixture of chemical compounds under conditions which permit the interaction of the chemical compound or the mixture of chemical compounds with the polypeptide,
(b) comparing the biological activity of the polypeptide in the presence of the chemical compound or the mixture of chemical compounds with the biological activity of the polypeptide in the absence of the chemical compound or the mixture of chemical compounds, or
(c) determining binding of the chemical compound or the mixture of chemical compounds to the polypeptide and
(d) determining the chemical compound which specifically modulates the biological activity of the polypeptide or binds to the polypeptide, thereby identifying the inhibitor.

13. The method according to claim 12, wherein said polypeptide comprises the amino acid sequence of SEQ ID NO:2.

14. Use of an inhibitor obtained by the method according to any of claims 10 to 13.

15. Herbicidal composition comprising an inhibitor according to claim 14.


SEQUENCE LISTING

<110> Bayer CropScience GmbH
      Max-Planck-Gesellschaft zur Förderung der Wissenschaften e.V.

<120> Lipid metabolic and signalling pathways in the epidermis

<130> GI-014 EP

<140> unknown

<141> 2004-08-09

<160> 7

<170> PatentIn version 3.1

<210> 1
<211> 1785
<212> DNA
<213> Arabidopsis thaliana

<400> 1
atggctctca agctctttct ctttgctctt cttctctgtc tcccgacttc tctctcctcc      60

actgcctcta aaggtaaaga gaagaagtcg aagtttaatc catacagata cacattcatc     120

gataaagcaa gcacattctc atcatcttcc tcatcttcat tttcatccaa cggtcaagat     180

tcgtcctacg actacatagt catcggaggt ggaaccgcag ggtgtcctct cgccgcaacg     240

ttgtcgcaga atttcagcgt tcttgtttta gagagaggtg gcgttccgtt tacaaacgca     300

aacgtttctt tcctcaggaa ttttcacatc ggacttgctg acatttcagc ttcttccgcg     360

tctcaagcgt ttgtttccac tgacggcgtt tacaacgccc gtgctagagt tctcggtggc     420

ggttcctgta ttaacgccgg tttttactcc agagccgatg ctgcgttcgt gaagcgagca     480

ggatgggatc cgaagctggt gaaggagtcg tatccatggg tggagagaga gattgttcat     540

cagccaaagt taacgttatg gcagaaagct ctcagagaca gtcttttaga ggttggagtc     600

agacctttca atggtttcac ttacgatcac gtttccggaa ccaaaatcgg cggtacaatt     660

ttcgacagat tcggccgtcg tcacaccgcg gcggagcttc tcgcttacgc taatcctcag     720

aagcttagag tcttgatcta cgccaccgtg caaaaaatcg tctttgacac ttctggaaca     780

aggcctcgag taacaggagt aatattcaaa gatgagaaag gtaatcaaca ccaggcttta     840

ctctcgaata gaaagggaag tgaagtgatc ttatctagtg gagctattgg gtcaccacag     900

atgctgatgt taagtgggat tggacctaag aaggagcttc agaggctgaa gattcctgtg     960

gttttagaga atgagcatgt aggaaaagga atggctgata atcccatgaa cacgatcttg    1020

gtgccttcaa aggcgcctat agagcagtca cttattcaga ctgttggaat tacaaagatg 1080

ggtgtgtatg ttgaagccag tactggcttt gggcaatctc ctgagagtat tcatactcac 1140

tatgggatta tgtcgaacaa gaatgaattg ttttccacca ttcctgcaaa gcagagaaga 1200

ccagaagcaa cgcaagctta catcacaaga aacaaatacc aacttcacga agcattcaat 1260

ggaagtttca tcttggagaa acttgcttac ccgatctcta gagggcattt gagcttggtc 1320

aacacaaatg ttgatgacaa cccttcagtc accttcaatt actttaaaca cccggtggat 1380

ctccaacgct gtgttgaagc cattcgtctt gtttccaaag tggtgacgtc taaccgtttc 1440

ttaaactaca cgcagtgtga caagcaaaac gtacacaaga tgcttagctt aagcgtcaag 1500

gcaaacatca atctaaggcc aaagcaactg aacgatacca aatcaatggc tcagttctgc 1560

aaagacactg ttgtcacaat ctggcactac catggtggat gtcttgtggg taaagttgtg 1620

agccctaacc gcaaagttct tggtgtcgac aggctcagag ttattgatgg ttcaacgttt 1680

gacgagtctc caggaaccaa cccgcaagct actatgatga tgatgggaag atacatggga 1740

gtcaagattc ttcgggagag acttggaaac aaagctggtg tttag 1785

<210> 2
<211> 594
<212> PRT
<213> Arabidopsis thaliana

<400> 2
Met Ala Leu Lys Leu Phe Leu Phe Ala Leu Leu Leu Cys Leu Pro Thr
1               5                   10                  15

Ser Leu Ser Ser Thr Ala Ser Lys Gly Lys Glu Lys Lys Ser Lys Phe
            20                  25                  30

Asn Pro Tyr Arg Tyr Thr Phe Ile Asp Lys Ala Ser Thr Phe Ser Ser
        35                  40                  45

Ser Ser Ser Ser Ser Phe Ser Ser Asn Gly Gln Asp Ser Ser Tyr Asp
    50                  55                  60

Tyr Ile Val Ile Gly Gly Gly Thr Ala Gly Cys Pro Leu Ala Ala Thr
65                  70                  75                  80

Leu Ser Gln Asn Phe Ser Val Leu Val Leu Glu Arg Gly Gly Val Pro
                85                  90                  95

Phe Thr Asn Ala Asn Val Ser Phe Leu Arg Asn Phe His Ile Gly Leu
            100                 105                 110

Ala Asp Ile Ser Ala Ser Ser Ala Ser Gln Ala Phe Val Ser Thr Asp
        115                 120                 125

Gly Val Tyr Asn Ala Arg Ala Arg Val Leu Gly Gly Gly Ser Cys Ile
    130                 135                 140

Asn Ala Gly Phe Tyr Ser Arg Ala Asp Ala Ala Phe Val Lys Arg Ala
145                 150                 155                 160

Gly Trp Asp Pro Lys Leu Val Lys Glu Ser Tyr Pro Trp Val Glu Arg
                165                 170                 175

Glu Ile Val His Gln Pro Lys Leu Thr Leu Trp Gln Lys Ala Leu Arg
            180                 185                 190

Asp Ser Leu Leu Glu Val Gly Val Arg Pro Phe Asn Gly Phe Thr Tyr
        195                 200                 205

Asp His Val Ser Gly Thr Lys Ile Gly Gly Thr Ile Phe Asp Arg Phe
    210                 215                 220

Gly Arg Arg His Thr Ala Ala Glu Leu Leu Ala Tyr Ala Asn Pro Gln
225                 230                 235                 240

Lys Leu Arg Val Leu Ile Tyr Ala Thr Val Gln Lys Ile Val Phe Asp
                245                 250                 255

Thr Ser Gly Thr Arg Pro Arg Val Thr Gly Val Ile Phe Lys Asp Glu
            260                 265                 270

Lys Gly Asn Gln His Gln Ala Leu Leu Ser Asn Arg Lys Gly Ser Glu
        275                 280                 285

Val Ile Leu Ser Ser Gly Ala Ile Gly Ser Pro Gln Met Leu Met Leu
    290                 295                 300

Ser Gly Ile Gly Pro Lys Lys Glu Leu Gln Arg Leu Lys Ile Pro Val
305                 310                 315                 320

Val Leu Glu Asn Glu His Val Gly Lys Gly Met Ala Asp Asn Pro Met
                325                 330                 335

Asn Thr Ile Leu Val Pro Ser Lys Ala Pro Ile Glu Gln Ser Leu Ile
            340                 345                 350

Gln Thr Val Gly Ile Thr Lys Met Gly Val Tyr Val Glu Ala Ser Thr
        355                 360                 365

Gly Phe Gly Gln Ser Pro Glu Ser Ile His Thr His Tyr Gly Ile Met
    370                 375                 380

Ser Asn Lys Asn Glu Leu Phe Ser Thr Ile Pro Ala Lys Gln Arg Arg
385                 390                 395                 400

Pro Glu Ala Thr Gln Ala Tyr Ile Thr Arg Asn Lys Tyr Gln Leu His
                405                 410                 415

Glu Ala Phe Asn Gly Ser Phe Ile Leu Glu Lys Leu Ala Tyr Pro Ile
            420                 425                 430

Ser Arg Gly His Leu Ser Leu Val Asn Thr Asn Val Asp Asp Asn Pro
        435                 440                 445

Ser Val Thr Phe Asn Tyr Phe Lys His Pro Val Asp Leu Gln Arg Cys
    450                 455                 460

Val Glu Ala Ile Arg Leu Val Ser Lys Val Val Thr Ser Lys Arg Phe
465                 470                 475                 480

Leu Asn Tyr Thr Gln Cys Asp Lys Gln Asn Val His Lys Met Leu Ser
                485                 490                 495

Leu Ser Val Lys Ala Asn Ile Asn Leu Arg Pro Lys Gln Leu Asn Asp
            500                 505                 510

Thr Lys Ser Met Ala Gln Phe Cys Lys Asp Thr Val Val Thr Ile Trp
        515                 520                 525

His Tyr His Gly Gly Cys Leu Val Gly Lys Val Val Ser Pro Asn Arg
    530                 535                 540

Lys Val Leu Gly Val Asp Arg Leu Arg Val Ile Asp Gly Ser Thr Phe
545                 550                 555                 560

Asp Glu Ser Pro Gly Thr Asn Pro Gln Ala Thr Met Met Met Met Gly
                565                 570                 575

Arg Tyr Met Gly Val Lys Ile Leu Arg Glu Arg Leu Gly Asn Lys Ala
            580                 585                 590

Gly Val

<210> 3
<211> 1942
<212> DNA
<213> Arabidopsis thaliana

<400> 3
caaaagagga ctaaagagga gagaaacaaa gaatcttctt actctccaaa ggtttgatct 60

ccagttcttg acgttttttt ctccttattc cctcactcat gagtccaaaa cgctgcgttt 120

atagggacct tttgctctgt tttattagaa cttctcatta tacacacgag tgaattagaa 180

cttgaaaacg ttgtggcatc atttgaatat ttttccttca aataaacaaa aaaaaaaaga 240

tgaagatgaa aaccaataat aaatatatgt acgttctata ataactcgtt tcatgaaagt 300

ggaagcatgg gatgagatgc caatcccatg aaaagtgagc aaaaattaat atttacatta 360

actgctgagt gggtaaagtt taataaccat tttctaaaaa taacttttat tcagaatcca 420

actcatggat tggtgtgtgt atttattatc atcatttcta tacacatatc cacccccaat 480

aactctgtca attatacacc tcaaaatcac tgtaagttat aaaaaaatga atttctacaa 540

ttaatcaaag accgtccaaa gccttcgtgg ctaattggtg ttaaactatt cacattttta 600

gtcaagtatt tttgcatatt atatgagatt aattttgcca cacaagatcg ttaataaact 660

cgacatgatg attctgttta tgtggcttct attaactagg aaatgaaaca atgtagttat 720

agttgtgact gatatgaagt cagagaaaat ggctacgtaa tcttatatga ccagaccaac 780

gtacgtgatg cgatgtgaca tttttcattc aacactaaag ttaaaacctt gattgttttt 840

gtattacatt tggcaactaa tccaatatgt tttgttagtt gttagtacta agtactgact 900

gatgatcaca ttcacgctca accaaataaa acggatgata ttgttttggg ttaggtgaaa 960

gaacaattaa attagttgcc aaaaacataa cccgtgaaga tttatgttag taaccaacct 1020

acagtcataa ttaataagtt tatgactaat agtaattaaa tgacttgcca aaaacatggc 1080

tctgtatagt atagttttta gattttggtt aggtgaagat gattaattga tacactatga 1140

atctatgaag acttgatcca acggctacca gatctccgac ttcaactgtt ttcatatcgc 1200

cattaatgat acagacaagc tgtactacta tcctttttat ctctttattg ttttatatga 1260

ttttacagta atactattac taagtactaa caaattgttt ttctttgcag ctggtcccaa 1320

aagtcaaaat catgggacaa aaaaacgagt tattattctt attatcatca ccaaggtaaa 1380

aaaatgtgaa aggaaatcct tttaaggctt agtttcaata taaatacaaa gatgttattt 1440

gatagtgatt gttctttgta acgaagtact tatcattaat ttcaatttgc aacaaacaat 1500

tctgtaacaa taagtctaat gtgaggaaag ttactagctg gtaaaataaa ttagtaggta 1560

actatttttt atgagtaaat gagggaacta ttaattgagt aagatgattc atatgtcctt 1620

ttcatttgct atattcgaca gaatgggaca cacctaaaat caaatttacg ttttagtgta 1680

gaccacccca acatctcttt attacacaac ttgtgttttt ttttactata tgattttttt 1740

accgaatgtt gttgtctaac caaaattcaa tgtaccgtat aagactataa gttatacaac 1800

tcgtagaaat ggttacatat atgtaacaat atatagagac acatgtgggt gtaattaata 1860

tagttgctct gttggatcgt tagtgttatt tataggagaa gaaaacacac aaaacaagac 1920

cattcatcaa aagccacata ag 1942

<210> 4
<211> 57
<212> DNA
<213> Artificial sequence

<220>
<223> Primer

<400> 4
ctcgagttta ggtgacacta tagaactgga gcctccactg cctctaaagg taaagag 57

<210> 5
<211> 52
<212> DNA
<213> Artificial sequence

<220>
<223> Primer

<400> 5
ctcgagtaat acgactcact atagggagac accaagaact ttgcggttag gg 52

<210> 6
<211> 38
<212> DNA
<213> Artificial sequence

<220>
<223> Primer

<400> 6
aaaaaaagct tcaaaagagg actaaagagg agagaaac 38


<210> 7
<211> 37
<212> DNA
<213> Artificial sequence

<220>
<223> Primer

<400> 7
aaaaaatcta gacttatgtg gcttttgatg aatggtc 37